# EUROPEAN PATENT SPECIFICATION

(11) **EP 2 836 077 B1**
(45) Date of publication and mention of the grant of the patent: **27.12.2017**
(21) Application number: 12720957.5
(22) Date of filing: 12.04.2012
(51) Int. Cl.: A23C 9/13, A23C 9/137

(54) **NEW FERMENTED DAIRY PRODUCT COMPRISING MICROCAPSULES AND PROCESS FOR PREPARING THE SAME**
NEUES FERMENTIERTES MILCHPRODUKT MIT MIKROKAPSELN UND VERFAHREN ZUR HERSTELLUNG DAVON
NOUVEAU PRODUIT LAITIER FERMENTÉ COMPRENANT DES MICROCAPSULES ET PROCÉDÉ POUR LE PRÉPARER

(43) Date of publication of application: 18.02.2015
(73) Proprietor: Compagnie Gervais Danone, 75009 Paris (FR); Danone, S.A., E-08029 Barcelona (ES)
(72) Inventor: COLIN, Cyril, F-92290 Chatenay-Malabry (FR); GASSET-PINON, Carolina, E-08034 Barcelona (ES); RODRIGUEZ, Eric, F-91520 Egly (FR)
(74) Representative: Regimbeau
(86) International application number: PCT/IB2012/000839
(87) International publication number: WO 2013/153414

(56) References cited:
- EP-A1- 1 342 548
- EP-A1- 1 836 902
- WO-A1-98/18610
- WO-A1-99/48386
- WO-A1-2009/005720
- WO-A1-2015/055775
- WO-A2-97/34496
- WO-A2-2007/102915
- US-A- 4 749 575
- MICHAEL M ET AL: "Impact of a plant extract on the viability of Lactobacillus delbrueckii ssp. bulgaricus and Streptococcus thermophilus in nonfat yogurt", INTERNATIONAL DAIRY JOURNAL, ELSEVIER APPLIED SCIENCE, BARKING, GB, vol. 20, no. 10, 1 October 2010 (2010-10-01), pages 665-672, XP027174042, ISSN: 0958-6946 [retrieved on 2010-04-07]

## Description

The present invention relates to the field of fermented dairy product and to its process of preparation. In particular, the present invention relates to a fermented dairy product comprising microcapsules of oxidizable active.

Products such as dairy fermented products are well known. They typically comprise milk or milk components (including water), fermented by lactic acid bacteria. Such products are available in various forms, including "yogurts" and "fermented milk products". Fermented milk products of the yoghurt type are obtained by fermentation of the milk with a combination of strains of lactic bacteria *Streptococcus thermophilus* and *Lactobacillus bulgaricus.* These products are available with various nutritional profiles depending typically on the amount of fat, protein, and carbohydrates, and added sugar, in the composition. The presence of lactic acid bacteria, and the fermentation typically allow proteins of milk or milk components to coagulate and thus to provide texture. The viscosity can then be adjusted by stirring. Moreover, the lactic acid bacteria have well known to have beneficial effects on health. It has been shown that some lactic bacteria, in particular lactobacilli and bifidobacteria improve immunity against infectious agents (Paubert-Braquet et al. Int J. Immunother. 11, 153-161 (1995)).

Known on the market is the existence of milk-based or milk-derived products that are supplemented with the addition of external substances. In particular in the dairy field, there are products derived from fermented milk, such as yoghurts, that are supplemented with the addition of a wide variety of foodstuffs selected from: minerals, vitamins, fruits and others. Therefore, the consumer could find products (beverage, milk product) containing supplemented vitamin C and it is still common to see nutritional labels food and beverage packages claiming to provide between 10% and 100% or more per serving of the daily value of vitamin C (60mg). Unfortunately, most if not all of these foods don't have the amount of vitamin C above mentioned at the end of the shelf life. These products don't guarantee to the consumer, the minimum daily requirements of vitamin C during all the storage of the product, and in particular at the end of the shelf life of the product (at least 35 days).

Vitamin C (ascorbic acid) is a water soluble vitamin that must be provided in the human diet because it is not synthesized by the body. Vitamin C or L-ascorbic acid or L-ascorbate is an essential nutrient for humans and certain other animal species. It is physiologically required for synthesis of many essential tissues as well as biomolecules including neurotransmitters, fat transport molecules and for catabolism of a portion of the body's cholesterol. Vitamin C is also an effective antioxidant in vivo for protecting many proteins, fats, carbohydrates and nucleic acids from damaging reactive oxygen and free radical species. For all these reasons, it is important to have products which can provide a daily intake of vitamin C. In addition to its benefits as a nutritional value in food products, ascorbic acid is also used extensively in food industry for its many functional contributions to product quality. Acting as an antioxidant, ascorbic acid can improve the color and palatability of many kinds of food products. By removing oxygen from its surroundings ascorbic acid in its reduced form becomes the oxidized form, dehydroascorbic acid. This oxidizing action reduces the available oxygen in its immediate environment, making ascorbic acid an effective antioxidant.

If vitamin C is added as a nutrient to food product, the stability of the vitamin C over the shelf-life of the products becomes important. During the preservation, in particular storage and transportation of products with vitamin C, there is a strong loss of vitamin C. Oxygen is the most destructive ingredient causing degradation of vitamin C. Publication of Gliguem et al. (J. Dairy Sci. 88: 891-899, 2005) describe processing and storage factors affecting the stability of vitamin C and its levels persisting in milk. These include the sterilization process, extent of oxygen and light penetration of the packaging, and the duration of storage. In conclusion, only packaging comprising an oxygen and light barrier is compatible with vitamin C fortification of milk. Furthermore, short storage time or low storage temperature is needed to retard vitamin C degradation. There is a need for a dairy product having a low rate of loss of vitamin C during a long period of storage (at least 35 days), regardless the packaging used.

The problem of the oxidation of vitamin C during storage could be solved by adding a compensating concentration before the process, such that the recommended level is still present at the end of the shelf life. However, the degradation of high amounts of vitamin C in milk could have some deleterious effects on other aspects of the nutritional quality (Birlouez-Aragon et al. 2004).

Up to now, number efforts have been reported to slow the degradation of ascorbic acid. For examples: reducing the availability of oxygen, reducing the pH of the composition or adding oxygen scavengers. Several chemistries that might help stabilize vitamin C have also been investigated to some degree, including mixed phase emulsions and sacrificial phenolic antioxydants.

US2011/0217410 describes foods and beverages with vitamin C in a manner such that vitamin C dissolved in an aqueous solution or suspension is protected against oxidative degradation. This involves the joint inclusion of a dissolved water-soluble vitamin C and a water insoluble chemical derivative of vitamin C in the aqueous medium. The water insoluble chemical derivative of vitamin C is ascorbyl palmitate, in particular microparticulate form of ascorbyl palmitate. This document shows that the vitamin C can be stabilized against oxidation in an aqueous medium by adding water-insoluble microparticulate ascorbyl palmitate, where the microparticulate ascorbyl palmitate is maintained in a substantially undissolved state, as a suspension in the product. The product does not describe microparticles of vitamin C but particles of ascorbyl palmitate. Moreover, this document does not describe a milk composition, in particular a fermented milk composition with at least a *Lactobacillus casei* strain and a *Streptococcus thermophilus* strain.

KR20040048749 discloses iron fortified milk which contains vitamin C microencapsulated by polyglycerol monostearate, preventing oxidation of iron.

WO9734496 and WO9948386 both describe yoghurt or yoghurt-like products fortified with encapsulated Vitamin C. In these documents, other encapsulating agents than those of the present invention are used. There is a need to make available a fermented dairy product providing the recommended daily allowances (RDA) of vitamin C at the end of the shelf life in particular at 35 days. Moreover, there is a need of a fermented dairy product in which the amount of viable lactic bacteria is maintained until the end of the shelf life. Typically, probiotics are live bacteria or active fractions thereof, that provide a health benefit to a host upon consumption. The inventors have surprisingly found that the *Streptococcus thermophilus* count decrease in contact with vitamin C. However, it is well known in the art that ascorbic acid tend to improve the viability of probiotic bacteria of the yogurt (Rajiv I. Dave et al. Int Dairy Journal 7 (1997) 435-443).

Hence, it is desirable to provide a fermented dairy product providing from 15% and 100% of RDA (Recommended dietary allowances) of oxidizable active, in particular vitamin C until the end of the shelf life of the product, having good organoleptic properties (no off-tastes, good mouth feel, no granular aspect in mouth...) and containing a sufficient level of viable lactic bacteria.

The present invention addresses at least one of the problems or needs above mentioned with a fermented dairy product comprising microcapsules of Vitamin C as the oxidizable active, and a fermented milk composition.

The present invention particularly relates to a fermented dairy product comprising microcapsules of Vitamin C and a fermented milk composition wherein the said fermented milk composition comprises at least a *Streptococcus thermophilus* strain.

The microcapsules according to the invention are microcapsules of Vitamin C comprise a core and a coating, said coating surrounding the core. The term "coating" used in the present invention means any layer (or coating layer) surrounding the core, once the encapsulating composition is applied and dried. In the present invention, the"core" comprises or consists in Vitamin C. The term "oxidizable" herein designates an agent which is able to become oxidized in contact with oxygen, in other words which is capable of undergoing a chemical reaction with oxygen. The term "active" "agent" "substance" can be used interchangeably. Outside the scope of the claimed invention, the oxidizable active is selected from the group comprising vitamin C, vitamin B5, vitamin B6, vitamin B8, vitamin B9, vitamin A, vitamin D3, vitamin K and vitamin E or a mixture thereof.

In the claimed invention, the oxidizable active is vitamin C, in particular natural vitamin C or synthetic vitamin C. The term "synthetic vitamin C" used in the present invention designates salts of vitamin C. The term "natural vitamin C" designates fruit or vegetable preparation comprising vitamin C or a mixture of fruit and vegetable preparation comprising vitamin C, which is optionally dried.

In a particularly embodiment, the vitamin C is salts of L. ascorbic acid, in particular sodium L-ascorbate, calcium L-ascorbate and iron L-ascorbate

In another embodiment, the vitamin C is a fruit juice comprising vitamin C in particular acerola juice, camu camu juice, rosehip juice, goji berry juice, blackcurrants juice, kiwi juice, orange juice, cranberries juice, bananas juice, pears juice, pomegranate juice, apples juice, grapes juice and mixtures thereof. The fruit juice can be a liquid juice, a concentrated or dried juice. The fruit juice can also comprises additives like starch and its derivatives, pectin, agar agar, carraghenans, gums or polysaccharides. Outside the scope of the claimed invention, the coating comprises or consists in an encapsulating composition selected from the group comprising or consisting of alginate, salts of alginate, starch, modified starch, sources of celluloses, cellulose gum, maltodextrin, ascorbyl palmitate, sugars, gelatins, polysaccharides, proteins and mixtures thereof. In the claimed invention, the encapsulating composition comprises alginate and ascorbyl palmitate. Again outside the scope of the claimed invention, the encapsulating composition comprises at least a modified starch, alginate and cellulose gum, optionally in combination with maltodextrin.

In an advantageous embodiment, the microcapsules of Vitamin C have an average diameter lower than 200 µm, in particular lower than 100 µm, more particularly lower than 50 µm. The term « average diameter » refers to the volume medium diameter D (v;0,5) meaning that approximately 50 volume % of the microcapsules have an equivalent spherical diameter that is smaller than the average diameter and approximately 50 volume % of the microcapsules have an equivalent spherical diameter that is greater than the average diameter. Particularly, the fermented diary product according to the invention is characterized in that 90% of microcapsules have a diameter lower than 200 µm, more particularly 70% of microcapsules have a diameter between 80 and 150 µm and 30% of microcapsules have a diameter between 150 and 200 µm.

In an advantageous embodiment, the microcapsules of Vitamin C are in a fruit preparation or a technological mass. According to the invention, the term "fruit preparation" designates fruit juice or fruit puree with or without pieces of fruits as the same kind of fruit than the puree or different kind of fruit. The term "fruit puree" or "fruit compote" can be used interchangeably. The term "technological mass" used in the invention describes a liquid composition without fruits or fruit juices, such as water. The fruit preparation and the technological mass can comprise or contain stabilizers such as alginate, starch, modified starch, sources of celluloses, cellulose gum, maltodextrin, ascorbyl palmitate, sugars, gelatins, polysaccharides, proteins and mixtures thereof and/or one or more ingredients selected among vitamins and/or minerals, said vitamins or minerals are selected from the group consisting of beta-carotene, vitamin A, vitamin B1, vitamin B2, vitamin B6, vitamin B12, vitamin D3, vitamin E, calcium, magnesium, iron, iodine, copper, manganese, potassium, chromium, molybdenum, boron, betaine, glutamic acid.

In a particularly embodiment, the amount of vitamin C can represent between 15% and 150% of RDA (Recommended dietary allowances), more particularly between 30% and 80% of RDA, even more particularly between 40% and 70% of RDA.

In an advantageous embodiment, 100g of said fermented dairy product comprises from 12 to 50 mg of vitamin C, in particular from 24 to 40 mg, more particularly from 30 to 35 mg of vitamin C, at the end of the shelf life, in particular up to 45 days, more particularly up to at 35 days. The product of the invention at the end of the process can contain for 100 g of said fermented dairy product, from 30 to 70 mg of vitamin C, in particular from 33 to 62 mg, of vitamin C.

In another embodiment, the rate of loss of vitamin C in said product is lower than 40%, in particular lower than 35%, even more particularly lower than 25%.

In a particularly embodiment, the fermented dairy product according to the invention comprises from 1.10⁵ to 1.10⁸ CFU/ml, more particularly from 8.10⁶ to 5.10⁷ CFU/ml of *Streptococcus thermophilus* strain, in particular at the end of the shelf life of the product. In a more preferred embodiment, *Streptococcus thermophilus* strain is selected among the strains which were deposited at the CNCM under the reference 1-2773, I-2778 and 1-2835.

In a particularly embodiment, the fermented dairy product according to the invention comprises from 1.10⁵ to 1.10⁹ CFU/ml of *Lactobacillus casei* strain. According to the invention, *Lactobacillus casei* strain is selected among the *Lactobacillus casei* ssp. *paracasei* strain or the *Lactobacillus paracasei* ssp. *paracasei* strain. The said *Lactobacillus casei* ssp. *paracasei* strain can be the strain which was deposited at the CNCM under the reference I-1518.

The product of the invention can comprises at least *Streptococcus thermophilus* strain and/or at least *Lactobacillus casei* strain in the fermented milk composition.

The term "fermented milk composition" is a milk composition fermented with at least *Streptococcus thermophilus* strain and/or at least *Lactobacillus casei* strain. During the fermentation of the milk composition, the strains produce lactic acid and the number of lactic acid bacteria increases. Lactic acid bacteria and fermented products are well known by the one skilled in the art. The term "milk composition" according to the invention, means a milk product, a milk-based product or a milk-based food product. These compositions comprise milk or milk components, wherein the milk chemical composition is modified for example by fermentation. Herein a "milk based composition" encompassed "milk containing" composition. The milk and/or milk components are preferably cow milk. The fermented milk composition could be typically a fermented milk product or yogurt. The term "fermented milks" or "yogurts" have the usual meanings attributed to them in the dairy industry, i.e products which are intended for animal consumption, more particularly human consumption, and which are derived from acidifying lactic fermentation of a dairy substrate (animal milk, in particular cow milk). More particularly the denomination "fermented milk" (degree n. deg 88.-1203 of December 30th, 1988) is reserved for a dairy product prepared with skimmed milk or not, or condensed milks or powders some, having undergone a heat treatment at least equivalent to pasteurization, and sown with producing micro-organisms of lactic acid such as the lactobacilli *(Lactobacillus acidophilus, L. casei, L. plantarum, L. reuteri, L. johnsonii*), the certain streptococci (*Streptococcus thermophilus*), bifidobacteria and the lactococci ones. Moreover, the term "yogurt" is reserved for the fermented milk obtained, using standard methods, by the development of specific thermophilic lactic bacteria designated *Lactobacillus bulgaricus* (also designated *Lactobacillus delbrueckii subsp. Bulgaricus*) and *Streptococcus thermophilus,* which must be alive in the finished product, in an amount of at least 1.10⁷ CFU/ml of S. thermophilus and *L. bulgaricus* strain per gram of product.

In a particularly embodiment, the fermented milk composition comprises at least one bacteria selected from the genera *Lactobacillus, Streptococcus, Lactococcus,* and *Bifidobacterium.*

In a preferred embodiment, the fermented milk composition comprises *Lactobacillus bulgaricus.*

In another embodiment, the fermented dairy product according to the invention comprises one or more ingredients selected among vitamins and/or minerals, said vitamins being not encapsulated, said ingredients can be selected from the group consisting of beta-carotene, vitamin A, vitamin B1, vitamin B2, vitamin B6, vitamin B12, vitamin D3, vitamin E, calcium, magnesium, iron, iodine, copper, manganese, potassium, chromium, molybdenum, boron, betaine, glutamic acid.

In a preferred embodiment, the fermented dairy product comprises vitamin D3 and/or vitamin B6. Vitamin B6, or pyridoxine is a component of the vitamin B complex and plays several physiologic roles, in particular in formation and health of red blood cells and blood vessels, nerve function, teeth or in amino acid metabolism or antioxidant properties. Vitamin D3 or cholecalciferol is a component of the vitamin D complex.

In a preferred embodiment, 100g of said fermented dairy product comprises from 0,5 to 15 µg of vitamin D3, in particular from 0,6 to 10 µg, more particularly from 0,7 to 4 µg of vitamin D3.

In more preferred embodiment of the present invention, 100g of said fermented dairy product comprises 0,85 µg of vitamin D3. In a more advantageous embodiment, the amount of vitamin D3 can represent 15 % or 20% or 25% or 50% or 70% or even 100% of RDA (Recomanded dietary allowances).

In a preferred embodiment, 100g of said fermented dairy product comprises from 0,05 to 2,5 mg of vitamin B6, in particular from 0,08 to 2 mg, more particularly from 0,12 to 1,5 mg of vitamin B6. In an advantageous embodiment of the present invention, 100g of said fermented dairy product comprises 0,15 mg of vitamin B6. In a more advantageous embodiment, the amount of vitamin B6 can represent 15 % or 20% or 25% or 50% or 70% or even 100% of RDA (Recomanded dietary allowances).

In an advantageous embodiment, the fermented dairy product according to the invention comprises one or more additives selected from sweeteners, coloring agents, flavors, flavor enhancers, sugars, preservatives and combinations thereof.

In a preferred embodiment, the fermented dairy product according to the invention comprises:
- from 0,005 to 5 % of microcapsules of vitamin C by weight of product, in particular from 0,015 to 3,5%, more particularly from 0.2 to 2% of microcapsules of vitamin C by weight of product, and
- from 1% to 99,95 % of fermented milk composition by weight of product, in particular from 10 to 80%, more particularly from 50 to 70% of fermented milk composition by weight of product, wherein the said fermented milk composition comprises a least *Lactobacillus casei* strain and/or *Streptococcus thermophilus* strain.

In a more preferred embodiment, the fermented dairy product according to the invention comprises from 0,01 to 5%, in particular from 0,005 to 1% of microcapsules of vitamin C by weight of fruit preparation or of technological mass.

In another embodiment, the fermented dairy product according to the invention comprises from 5 % to 90% of orange juice or technological mass comprising from 0.01% to 5% of microcapsules of vitamin C by weight of juice or technological mass.

The present invention also relates to a method comprising the step of dispersing of microcapsules Vitamin c with a fermented milk composition.

In another embodiment of the present invention, the method comprises:
a) a step of fermentation of a milk composition with *Lactobacillus casei* and *Streptococcus thermophilus* to obtain a fermented milk composition, and
b) a step of mixing of the fermented milk composition obtained in step a) with at least 0,005 to 5 % by weight of the product, of microcapsules of Vitamin C.
In another embodiment, in the step b) of the method the fermented milk composition obtained in step a) is mixed with microcapsules of vitamin C, in particular is mixed with a fruit preparation comprising microcapsules of vitamin C or a technological mass comprising microcapsules of vitamin C.

In a preferred embodiment, the method according to the invention wherein step b) comprises the following steps:
- b1) preparing microcapsules of vitamin C,
- b2) mixing microcapsules obtained in step b1) with a fruit preparation or a technological mass,
- b3) pasteurizing at a temperature of at least 85°C during 11 min,
- b4) mixing the pasteurized composition of microcapsules of vitamin C obtained in step b3) in the fermented milk composition obtained in step a).

In a more preferred embodiment, in the method according to the invention, the step b) is necessary after the step a), more particularly after an optionally step of homogenization.

### FIGURES

This invention is illustrated by the following figures:
FIGURE 1: figure 1 represents an optical microscopy view of the microcapsules C in the fermented dairy product C.
FIGURE 2: figure 2 represents an optical microscopy view of the microcapsules A in the fermented dairy product A.
FIGURE 3: figure 3 shows the vitamin C relative stability (%) of microcapsules C in the fermented dairy product C.

Further details or advantages of the invention might appear in the following non limitative examples.

### EXAMPLES

### EXAMPLE 1 Preparation of the product according to the invention

### 1) Preparation of microcapsules

Microcapsules of vitamin C were prepared by a step of spraying on particles consisting in vitamin C (sodium ascorbate), or particles comprising vitamin C (acerola juice), with the encapsulating compositions below mentioned. Then the microcapsules are dried to obtain dried microcapsules. 3 kinds of microcapsules of vitamin C were prepared either from acerola juice (Table 1) or from sodium ascorbate (Table 2), wherein the microcapsules A and microcapsules B are comparative to microcapsules C accodring to the claimed invention.

**Ex : Table 1: microcapsules of natural vitamin C**

| **Ingredients : Microcapsules A** | **g/100g of dried capsule** |
|---|---|
| Acerola juice | 60-65 |
| Modified starch | 20-30 |
| Maltodextrin | 5-20 |
| Alginate | 3-10 |
| Cellulose gum | 1-5 |

**Table 2: microcapsules of synthetic vitamin C with 2 different encapsulating compositions**

| **Ingredients** : **Microcapsules B** | **g/100g of dried capsule** |
|---|---|
| Sodium ascorbate | 60-65 |
| Modified amidon | 25-33 |
| Alginate | 5-10 |
| Cellulose gum | 1-5 |
| | |

| **Ingredients** : **Microcapsules C** | **g/100g of dried capsule** |
|---|---|
| Sodium ascorbate | 90-97 |
| Alginate | 3-10 |
| Ascorbyl palmitate | 0,2-1 |

### 2) Preparation of a fermented milk composition

Milk fermented with *Lactobacillus casei* strain and *Streptococcus thermophilus* strains was prepared. The *Lactobacillus casei* used is *Lactobacillus casei* CNCM I-1518. The *Streptococcus thermophilus* used are CNCM 1-2773, CNCM 1-2835, CNCM 1-2778. The fermentation step is well known by the one skilled in the art. The preparation of the fermented milk composition is standard for the other steps of preparation.

### 3) Preparation of fermented dairy products A, B and C according to the invention, comprising comparative microcapsules A and B and microcapsules C according to the claimed invention

Microcapsules A, B and C are prepared and mixed in a fruit preparation, in particular orange juice. The orange juice with microcapsules is pasteurized at a temperature of at least 85°C during 11 min, then the juice is cooled and storage in cool conditions before the mixing step. Then, 12% orange juice is added in a fermented milk composition.

### EXAMPLE 2: Analysis of vitamin C microcapsules in optical microscopy in the fermented dairy products A and C.

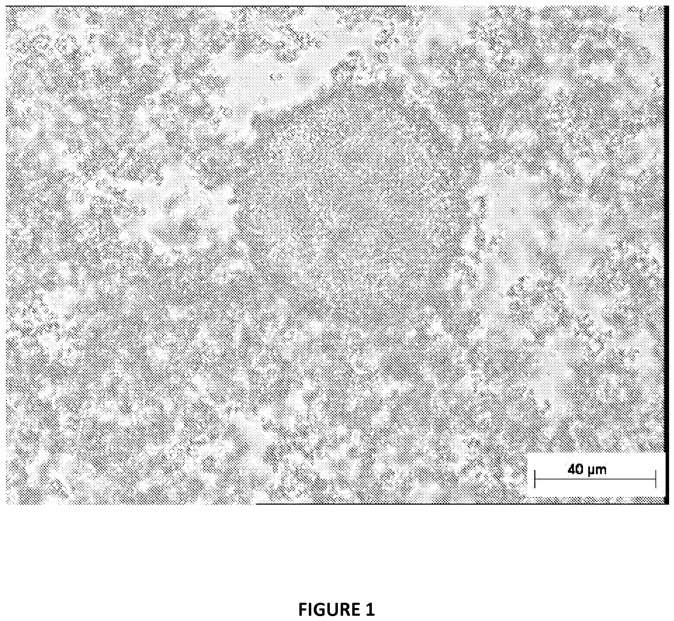

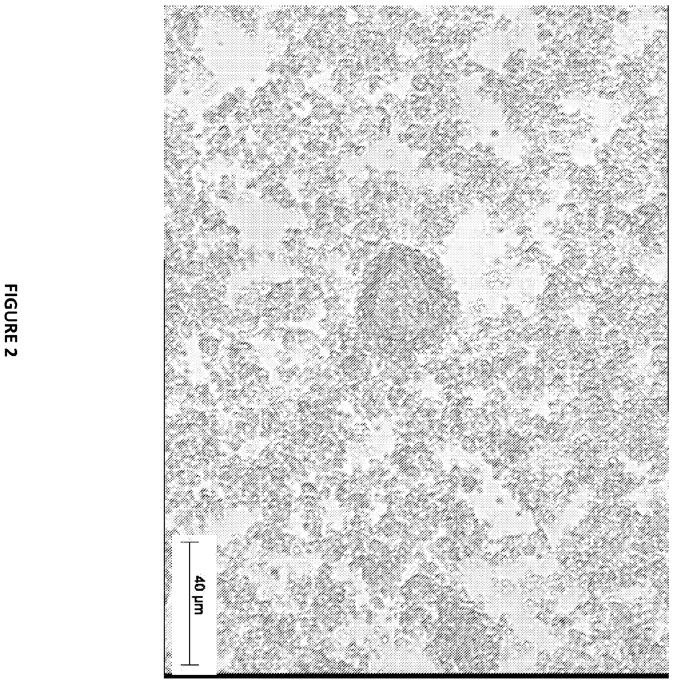

FIGURES 1 and 2 represent an optical microscopy view of the microcapsules C and A in the fermented dairy product C and A.

From the Figures 1 and 2, it can be observed that the coating according to the invention surrounding regularly the core ensuring a good stability of the vitamin C during the time and a good protection of the elements of the fermented milk composition in contact with these microcapsules.

These characteristics allow a long packaging time of the fermented dairy products A and C without significant degradation of its qualities, in particular organoleptic qualities.

### EXAMPLE 3: Study of the stability of vitamin C comparative microcapsules A, B and microcapsules C according to the claimed invention until the end of the shelf life

### a) Study of the stability of vitamin C comparative microcapsules A,B and microcapsules C according to the claimed invention until the end of the shelf life

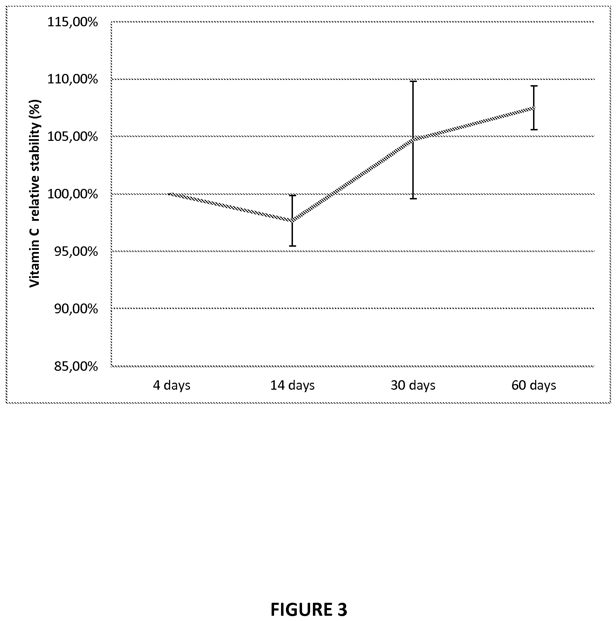

Stability of microcapsule C was analyzed during the storage of microcapsules up to 60 days. Figure 3 shows that during the storage time, microcapsules have a good stability when suspended in a dairy product (HPLC method standard deviation: ±10%; NF-V0135).

### b) Study of the stability of vitamin C microcapsules A, B and C in the fermented dairy products A, B and C until the end of the shelf life

The stability is determined by the measurement of the rate of vitamin C for each of the products A, B and C, the day of the production (D3) and 35 days after (D35), using the High Performance Liquid Chromatography. The rate of loss of vitamin C corresponds to the report of the amount of vitamin C at D35 versus the amount of vitamin C at D3.

Stability of microcapsules A, B and C was analyzed in fermented dairy products A, B and C Microcapsules of synthetic vitamin C comparative microcapsules B and microcapsules C according to the claimed invention or of natural vitamin C comparative microcapsule
A) were incorporated in the dairy products as of 80% RDA¹ or 30% RDA respectively. Results are the expression of at least three experiments.
i) Evaluation of the stability of the synthetic vitamin C microencapsulated and vitamin C non encapsulated (control)

| | microcapsule C | microcapsule B | non encapsulated |
|---|---|---|---|
| vitamin C D35/D3² (% of relative stability) | 73.18^{*} | 59.12^{*} | 27.53 |
| Standard deviation | 11.27 | 13.36 | 11.88 |

| | | | |
|---|---|---|---|
| ¹ RDA: EU 100% RDA is 80mg ; ²vitamin C was analyzed using HPLC method at D3 and D35 ^{*}significant versus control (no capsules) | | | |

At 80% RDA, results demonstrated that microcapsules C and comparative microcapsules B ensure significant synthetic vitamin C stability when compared to the control
i) Evaluation of the stability of the natural vitamin C microencapsulated and vitamin C non encapsulated (control)

| | Microcapsules A | non encapsulated |
|---|---|---|
| vitamin C D35/D3² (% of relative stability) | 67.15^{*} | 20.21 |
| Standard deviation | 6.5 | 3.4 |

| | | |
|---|---|---|
| ¹RDA: EU 100% RDA is 80mg ; ²vitamin C was analyzed using HPLC method at D3 and D35; ^{*}significant versus control (no capsules) | | |

At 30% RDA, results demonstrated that microcapsules A ensure a significant synthetic vitamin C stability when compared to the control

### EXAMPLE 4: Study of the impact of the vitamin C on the count of Streptococcus thermophilus in fermented dairy products

Microcapsules of synthetic (C and D) or natural vitamin C (A) were incorporated in the dairy products as of 80% RDA¹ or 30% RDA respectively. *Streptococcus thermophilus* counts were analyzed at D4, D14 and D35. Results are the expression of at least three experiments.
ii) Evaluation of the *Streptococcus thermophilus* counts stability during the product shelf-life (35days) when using microcapsules of synthetic vitamin C (C and D) and vitamin C non encapsulated (control)

| Cfu/ml of product | Microcapsules C | Microcapsules D | non encapsulated |
|---|---|---|---|
| D4 | 5.28E+08 | 5.40E+08 | 4.50E+08 |
| D14 | 1.98E+08 | 2.10E+08 | 1.46E+08 |
| D35 | 7.96E+07^{*} | 9.24E+07^{*} | 3.22E+05 |

| | | | |
|---|---|---|---|
| ^{*}significant versus control (no capsules) | | | |

At 80% RDA, results demonstrated a significant stability of the *Streptococcus thermophilus* counts at the end of the shelf-life (35 days) when using microcapsules of synthetic vitamin C (microcapsules and D) compared to the control
ii) Evaluation of the *Streptococcus thermophilus* counts stability during the product shelf-life (35days) when using microcapsules of natural vitamin C comparative microcapsule A) and vitamin C non encapsulated (control)

| Cfu/ml of product | Microcapsules A | non encapsulated |
|---|---|---|
| D4 | 2.81E+08 | 3.93E+08 |
| D14 | 8.78E+07 | 3.61E+07 |
| D35 | 1.34E+07 | <E+05 |

| | | |
|---|---|---|
| ^{*}significant versus control (no capsules) | | |

At 30% RDA, results demonstrated a significant stability of the *Streptococcus thermophilus* counts at the end of the shelf-life (35 days) when using microcapsules of synthetic vitamin C comparative microcapsule A) compared to control.

## Claims

1. A fermented dairy product comprising:
- microcapsules of vitamin C, whose coating consists in an encapsulating composition comprising alginate and ascorbyl palmitate, and
- a fermented milk composition.

2. The fermented dairy product according to claim 1 comprising:
- said microcapsules of vitamin C, and
- a fermented milk composition wherein the said fermented milk composition comprises at least a *Streptococcus thermophilus* strain and at least a *Lactobacillus casei* strain.

3. The fermented dairy product according to anyone of claims 1 to 2, **characterized in that** the vitamin C is salts of L. ascorbic acid, in particular sodium L-ascorbate, calcium L-ascorbate and iron L-ascorbate.

4. The fermented dairy product according to claim 1 to 3, **characterized in that** the vitamin C is a fruit preparation comprising vitamin C in particular acerola juice, camu camu juice, rosehip juice, goji berry juice, blackcurrants juice, kiwi juice, orange juice, cranberries juice, bananas juice, pears juice, pomegranate juice, apples juice, grapes juice and mixtures thereof.

5. The fermented dairy product according to claim 4, **characterized in that** the fruit juice is a liquid juice or a concentrated or dried juice.

6. The fermented dairy product according to any one of claims 1 to 5, **characterized in that** the encapsulating composition consists in alginate and ascorbyl palmitate.

7. The fermented dairy product according to anyone of claims 1 to 6, wherein the microcapsules of vitamin C have an average diameter lower than 200 µm, in particular lower than 100 µm, more particularly lower than 50 µm.

8. The fermented dairy product according to anyone of claims 1 to 7, **characterized in that** 100g of said fermented dairy product comprises from 12 to 50 mg of vitamin C, in particular from 24 to 40 mg, more particularly from 30 to 35 mg of vitamin C, at the end of the shelf life.

9. The fermented dairy product according to anyone of claims 1 to 8, wherein said product comprises from 1.10⁵ to 1.10⁸ CFU/ml, more particularly from 8.10⁶ to 5.10⁷ CFU/ml of *Streptococcus thermophilus.*

10. The fermented dairy product according to anyone of claims 1 to 9 comprising:
- from 0,005 to 5 % of microcapsules of vitamin C by weight of product, in particular from 0,015 to 3,5%, more particularly from 0.2 to 2% of microcapsules of vitamin C by weight of product, and
- from 1% to 99,95 % of fermented milk composition by weight of product, in particular from 10 to 80%, more particularly from 50 to 70% of fermented milk composition by weight of product.

11. The fermented dairy product according to anyone of claims 1 to 10 comprising from 5 % to 90% of orange juice comprising from 0.01% to 5% of microcapsules of vitamin C by weight of juice.

12. A method for preparing a fermented dairy product as defined in claims 1 to 11, said method comprising the step of dispersing said microcapsules of vitamin C with a fermented milk composition.

13. The method according to claim 12, comprising:
a) a step of fermentation of a milk composition with *Lactobacillus casei* and *Streptococcus thermophilus* to obtain a fermented milk composition, and
b) a step of mixing of the fermented milk composition obtained in step a) with at least 0,005 to 5 % by weight of the product, of said microcapsules of vitamin C.

14. The method according to claim 13, wherein in step b) the fermented milk composition obtained in step a) is mixed with a fruit preparation comprising said microcapsules of vitamin C.

15. The method for preparing a fermented dairy product according to anyone of claims 13 to 14 , wherein step b) comprises the following steps:
- b1) preparing said microcapsules of vitamin C,
- b2) mixing microcapsules obtained in step b1) with a fruit preparation,
- b3) pasteurizing at a temperature of at least 85°C during 11 min,
- b4) mixing the pasteurized composition of microcapsules of vitamin C obtained in step b3) in the fermented milk composition obtained in step a).

## Patentansprüche

1. Fermentiertes Milchprodukt, das Folgendes umfasst
- Mikrokapseln mit Vitamin C, deren Beschichtung aus einer Einkapselungszusammensetzung besteht, die Alginat und Ascorbylpalmitat umfasst, und
- eine fermentierte Milchzusammensetzung.

2. Fermentiertes Milchprodukt nach Anspruch 1, das Folgendes umfasst:
- die Mikrokapseln mit Vitamin C, und
- eine fermentierte Milchzusammensetzung, wobei die fermentierte Milchzusammensetzung mindestens einen *Streptococcus-thermophilus*-Stamm und mindestens einen *Lactobacillus-casei*-Stamm umfasst.

3. Fermentiertes Milchprodukt nach einem der Ansprüche 1 bis 2, **dadurch gekennzeichnet, dass** das Vitamin C Salze von L-Ascorbinsäure, insbesondere Natrium-L-Ascorbat, Calcium-L-Ascorbat und Eisen-L-Ascorbat, ist.

4. Fermentiertes Milchprodukt nach Anspruch 1 bis 3, **dadurch gekennzeichnet, dass** das Vitamin C eine Fruchtzubereitung ist, die Vitamin C, insbesondere Acerolasaft, Camu-Camu-Saft, Hagebuttensaft, Gojibeerensaft, schwarze Johannisbeerensaft, Kiwisaft, Orangensaft, Moosbeerensaft, Bananensaft, Birnensaft, Granatapfelsaft, Apfelsaft, Traubensaft und Mischungen daraus, umfasst.

5. Fermentiertes Milchprodukt nach Anspruch 4, **dadurch gekennzeichnet, dass** der Fruchtsaft ein flüssiger Saft oder ein konzentrierter oder getrockneter Saft ist.

6. Fermentiertes Milchprodukt nach einem der Ansprüche 1 bis 5, **dadurch gekennzeichnet, dass** die Einkapselungszusammensetzung aus Alginat und Ascorbylpalmitat besteht.

7. Fermentiertes Milchprodukt nach einem der Ansprüche 1 bis 6, wobei die Mikrokapseln mit Vitamin C einen durchschnittlichen Durchmesser aufweisen, der kleiner als 200 µm, insbesondere kleiner als 100 µm, genauer gesagt kleiner als 50 µm ist.

8. Fermentiertes Milchprodukt nach einem der Ansprüche 1 bis 7, **dadurch gekennzeichnet, dass** 100 g des fermentierten Milchprodukts am Ende der Haltbarkeitsdauer von 12 bis 50 mg Vitamin C, insbesondere von 24 bis 40 mg, genauer gesagt von 30 bis 35 mg Vitamin C umfassen.

9. Fermentiertes Milchprodukt nach einem der Ansprüche 1 bis 8, wobei das Produkt zwischen 1,10⁵ und 1,10⁸ KBE/ml, insbesondere zwischen 8,10⁶ und 5,10⁷ KBU/ml *Streptococcus thermophilus* umfasst.

10. Fermentiertes Milchprodukt nach einem der Ansprüche 1 bis 9, das Folgendes umfasst:
- zwischen 0,005 und 5 Gew.-% des Produkts Mikrokapseln mit Vitamin C, insbesondere zwischen 0,015 und 3,5 %, genauer gesagt zwischen 0,2 und 2 Gew.-% des Produkts Mikrokapseln mit Vitamin C, und
- zwischen 1 % und 99,95 Gew.-% des Produkts fermentierte Milchzusammensetzung, insbesondere zwischen 10 und 80 %, genauer gesagt zwischen 50 und 70 Gew.-% des Produkts fermentierte Milchzusammensetzung.

11. Fermentiertes Milchprodukt nach einem der Ansprüche 1 bis 10, das zwischen 5 % und 90 % Orangensaft umfasst, der zwischen 0,01 und 5 Gew.-% des Safts Mikrokapseln mit Vitamin C umfasst.

12. Verfahren zur Herstellung eines fermentierten Milchprodukts nach Anspruch 1 bis 11, wobei das Verfahren den Schritt zum Verteilen der Mikrokapseln mit Vitamin C mit einer fermentierten Milchzusammensetzung umfasst.

13. Verfahren nach Anspruch 12, das Folgendes umfasst:
a) einen Schritt zum Fermentieren einer Milchzusammensetzung mit *Lactobacillus casei* und *Streptococcus thermophilus* zum Erhalten einer fermentierten Milchzusammensetzung, und
b) einen Schritt zum Mischen der im Schritt a) erhaltenen fermentierten Milchzusammensetzung mit mindestens 0,005 bis 5 Gew.-% des Produkts Mikrokapseln mit Vitamin C.

14. Verfahren nach Anspruch 13, wobei im Schritt b) die in Schritt a) erhaltene fermentierte Milchzusammensetzung mit einer Fruchtzubereitung gemischt wird, die die Mikrokapseln mit Vitamin C umfasst.

15. Verfahren zur Herstellung eines fermentierten Milchprodukts nach einem der Ansprüche 13 bis 14, wobei der Schritt b) die folgenden Schritte umfasst:
- b1) Herstellen der Mikrokapseln mit Vitamin C,
- b2) Mischen der im Schritt b1) erhaltenen Mikrokapseln mit einer Fruchtzubereitung,
- b3) Pasteurisieren bei einer Temperatur von mindestens 85 °C während 11 Minuten,
- b4) Mischen der im Schritt b3) erhaltenen pasteurisierten Zusammensetzung aus Mikrokapseln mit Vitamin C in der im Schritt a) erhaltenen fermentierten Milchzusammensetzung.

## Revendications

1. Produit laitier fermenté comprenant :
- des microcapsules de vitamine C, dont l'enrobage consiste en une composition d'encapsulation comprenant de l'alginate et du palmitate d'ascorbyle, et
- une composition de lait fermenté.

2. Produit laitier fermenté selon la revendication 1, comprenant :
- lesdites microcapsules de vitamine C, et
- une composition de lait fermenté, dans laquelle ladite composition de lait fermenté comprend au moins une souche de *Streptococcus thermophilus* et au moins une souche de *Lactobacillus casei.*

3. Produit laitier fermenté selon l'une quelconque des revendications 1 à 2, **caractérisé en ce que** la vitamine C est des sels d'acide L-ascorbique, en particulier le L-ascorbate de sodium, le L-ascorbate de calcium et le L-ascorbate de fer.

4. Produit laitier fermenté selon l'une quelconque des revendications 1 à 3, **caractérisé en ce que** la vitamine C est une préparation de fruits comprenant de la vitamine C, en particulier du jus d'acérola, du jus de camu camu, du jus de cynorrhodon, du jus de baies de goji, du jus de cassis, du jus de kiwi, du jus d'orange, du jus de canneberges, du jus de bananes, du jus de poires, du jus de grenade, du jus de pommes, du jus de raisin et des mélanges de ceux-ci.

5. Produit laitier fermenté selon la revendication 4, **caractérisé en ce que** le jus de fruit est un jus liquide ou un jus concentré ou séché.

6. Produit laitier fermenté selon l'une quelconque des revendications 1 à 5, **caractérisé en ce que** la composition d'encapsulation consiste en alginate et palmitate d'ascorbyle.

7. Produit laitier fermenté selon l'une quelconque des revendications 1 à 6, dans lequel les microcapsules de vitamine C ont un diamètre moyen inférieur à 200 µm, en particulier inférieur à 100 µm, plus particulièrement inférieur à 50 µm.

8. Produit laitier fermenté selon l'une quelconque des revendications 1 à 7, **caractérisé en ce que** 100 g du produit laitier fermenté comprend de 12 à 50 mg de vitamine C, en particulier de 24 à 40 mg, plus particulièrement de 30 à 35 mg de vitamine C, au terme de sa durée de vie.

9. Produit laitier fermenté selon l'une quelconque des revendications 1 à 8, dans lequel ledit produit comprend de 1,10⁵ à 1,10⁸ UFC/ml, plus particulièrement 8,10⁶ à 5,10⁷ UFC/ml de *Streptococcus thermophilus.*

10. Produit laitier fermenté selon l'une quelconque des revendications 1 à 9, comprenant :
- de 0,005 à 5 % de microcapsules de vitamine C en poids du produit, en particulier de 0,015 à 3,5 %, plus particulièrement de 0,2 à 2 % de microcapsules de vitamine C en poids de produit, et
- de 1 % à 99,95 % de composition de lait fermenté en poids de produit, en particulier de 10 à 80 %, plus particulièrement de 50 à 70 % de composition de lait fermenté en poids de produit.

11. Produit laitier fermenté selon l'une quelconque des revendications 1 à 10, comprenant de 5 % à 90 % de jus d'orange comprenant de 0,01 % à 5 % de microcapsules de vitamine C en poids de jus.

12. Procédé de préparation d'un produit laitier fermenté tel que défini selon les revendications 1 à 11, ledit procédé comprenant l'étape de dispersion desdites microcapsules de vitamine C avec une composition de lait fermenté.

13. Procédé selon la revendication 12, comprenant :
a) une étape de fermentation d'une composition de lait avec *Lactobacillus casei* et *Streptococcus thermophilus* pour obtenir une composition de lait fermenté, et
b) une étape de mélange de la composition de lait fermenté obtenue à l'étape a) avec au moins 0,005 à 5 % en poids du produit, desdites microcapsules de vitamine C.

14. Procédé selon la revendication 13, dans lequel, à l'étape b), la composition de lait fermenté obtenue à l'étape a) est mélangée avec une préparation de fruits comprenant lesdites microcapsules de vitamine C.

15. Procédé de préparation d'un produit laitier fermenté selon l'une quelconque des revendications 13 à 14, dans lequel l'étape b) comprend les étapes suivantes :
- b1) préparation desdites microcapsules de vitamine C,
- b2) mélange des microcapsules obtenues à l'étape b1) avec une préparation de fruits,
- b3) pasteurisation à une température d'au moins 85 °C pendant 11 min,
- b4) mélange de la composition pasteurisée de microcapsules de vitamine C obtenue à l'étape b3) dans la composition de lait fermenté obtenue à l'étape a).
